# EUROPEAN PATENT APPLICATION

(11) **EP 2 228 077 A2**
(43) Date of publication of application: **15.09.2010**
(21) Application number: 10250198.8
(22) Date of filing: 05.02.2010
(51) Int. Cl.: A61L 15/20, A61L 15/28, A61L 15/46

(54) **Wound dressing with antimicrobial gauze and bonding agent**

(30) Priority: 20.02.2009 US 390190
(71) Applicant: Medline Industries, Inc.,, Mundelein, IL 60060 (US)
(72) Inventor: Min, Yao, Vernon Hills, Illinois 60061 (US)
(74) Representative: McLeish, Nicholas Alistair Maxwell

(57) **Abstract**

A wound dressing (500) includes a flexible layer of material (501) that is treated with an antimicrobial agent (604), such as PHMB. The antimicrobial agent (604) ionically bonds to cellulose fibers (502) of the flexible layer of material (501) through a bonding agent (601). The bonding agent (601) increases the number of available carboxylate bonding groups beyond a characteristic carboxylate bonding group density normally associated with untreated cellulose fiber such that more antimicrobial agent (604) can be bonded to the flexible layer of material (501). An optional antifungal agent (606) can also be applied to the flexible layer of material (501).

## Description

### BACKGROUND

### TECHNICAL FIELD

The present invention generally relates to wound dressings and, more particularly, to a wound dressing in the form of gauze or another similar absorbent material having an antimicrobial agent, such as Polyhexamethylene biguanide (PHMB), where the antimicrobial agent is integrated with the gauze through an additional bonding agent.

### BACKGROUND ART

Wounds, such as cuts, lesions, and surgical incisions, are generally covered with a dressing, such as a bandage or gauze, to help them heal. Even where sutures or staples are used to close a wound, the wound is often covered with a dressing. Wound dressings help to absorb fluid emitted by the wound. Many wound dressings are porous in nature and are capable of both absorbing liquid from the wound and allowing oxygen to reach the wound, thereby assisting the healing process

One problem associated with wound dressings is that it is often difficult to prevent microbial growth under the dressing. For example, as most wound dressings are air-porous, airborne bacteria can migrate through the dressing and contact the wound. Further, the dressing may provide cover from light and temperature that permits cultivation of the bacteria. Additionally, the accumulation of material emitted by the wound, such as blood and wound fluids, can promote the growth of bacteria, thereby impeding the healing process.

There have been attempts to add antimicrobial additives to wound dressings to retard or prevent the growth of harmful bacteria near the wound. However, many of these antimicrobial dressings have shortcomings. For instance, they often have concentrations of antimicrobial additives that are less than effective in treating certain bacteria, such as pseudomonas aeruginosa.
Further, they are often constructed such that the antimicrobial agent is able to leach out and away from the dressing, thereby irritating the skin.

There is thus a need for an improved wound dressing with antimicrobial components that are more effective as biocides, yet that impede release of the antimicrobial components that may tend to irritate a patient's skin.

### BRIEF DESCRIPTION OF THE DRAWINGS

FIG. 1 illustrates a molecular diagram of a flexible layer of material having cellulose fiber in accordance with embodiments of the invention, both before and after textile processing.

FIG. 2 illustrates a molecular diagram of one antimicrobial agent in accordance with embodiments of the invention.

FIG. 3 illustrates a molecular diagram of an ether group formed when a flexible layer of material is treated with a bonding agent in accordance with embodiments of the invention.

FIG. 4 illustrates carboxlate groups formed with a flexible layer of material is treated with a bonding agent in accordance with embodiments of the invention.

FIG. 5 illustrates a wound dressing in accordance with embodiments of the invention.

FIG. 6 illustrates a process by which a wound dressing can be manufactured in accordance with embodiments of the invention.

FIG. 7 illustrates a wound dressing in accordance with embodiments of the invention.

FIG. 8 illustrates a method by which a wound dressing can be manufactured in accordance with embodiments of the invention.

FIG. 9 illustrates a method by which a wound may be treated in accordance with embodiments of the invention.

FIGS. 10 and 11 illustrate a process by which a wound dressing can be manufactured in accordance with embodiments of the invention.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of embodiments of the present invention.

### DETAILED DESCRIPTION OF THE INVENTION

Embodiments of the invention are now described in detail. Referring to the drawings, like numbers indicate like parts throughout the views. As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, and the like may be used solely to distinguish one entity or action from another entity or action without necessarily requiring or implying any actual such relationship or order between such entities or actions. Also, reference designators shown herein in parenthesis indicate components shown in a figure other than the one in discussion. For example, talking about a device (10) while discussing figure A would refer to an element, 10, shown in figure other than figure A.

Embodiments of the present invention provide a wound dressing that is treated with an antimicrobial agent. In one embodiment, the antimicrobial agent is PHMB. The PHMB is ionically bonded to the wound dressing by a bonding agent, such as chloroacetic acid or chlorocitric acid. The bonding agent increases the number of carboxyl bonding groups available to the antimicrobial agent, thereby permitting more of the antimicrobial agent to bond with the wound dressing than would be the case if the wound dressing were not treated with the bonding agent. In addition to permitting more of the antimicrobial agent to bond with the cellulose fiber of the wound dressing, the bonding agent helps to ensure that the antimicrobial agent remains bonded with the wound dressing. In so doing, the bonding agent inhibits leaching of the antimicrobial agent. As such, the bonding agent works to reduce or eliminate skin irritation when compared to prior art antimicrobial wound dressings.

In one embodiment, the wound dressing comprises a flexible layer of material that includes a cellulose fiber. For instance, in one embodiment, the cellulose fiber comprises cotton fibers. Cotton fibers are composed of linear cellulose polymer chains.

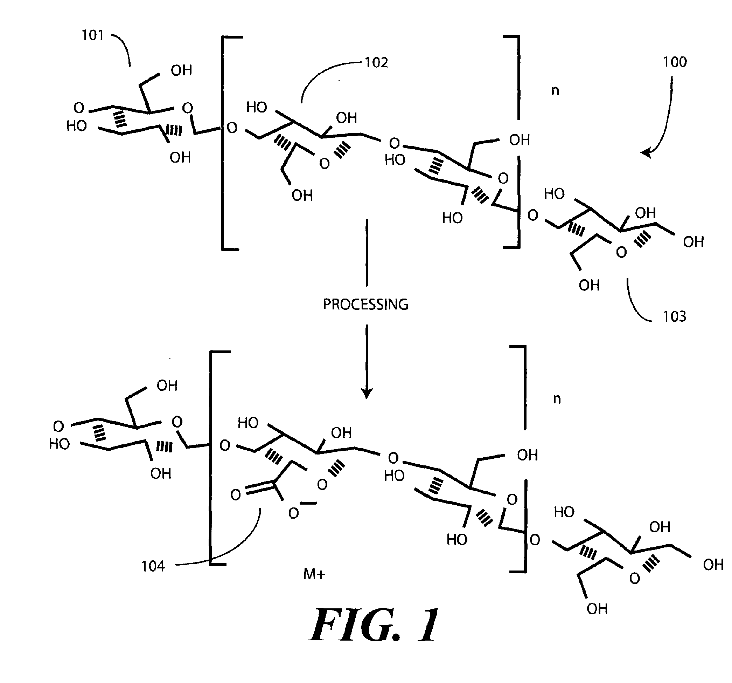

Turning now to FIG. 1, illustrated therein is an illustration of an exemplary cellulose polymer chain 100. Such a cellulose polymer chain 100 is found, for example, in surgical gauze made from cotton. The cellulose molecules 101,102,103 comprising the cellulose polymer chain 100 are composed of cellobiose units joined together by 1,4-β glycosidic bonds.

When material including the cellulose polymer chain 100 is processed, such as in the textile processing typically employed to make the material soft, sterile, and pliable, oxidation of the cellulose polymer chain 100 occurs. This oxidation, which commonly occurs, forms carboxylate groups (COO-) 104. Prior art solutions have attempted to attach antimicrobial agents to these carboxylate groups to yield an antimicrobial wound dressing.

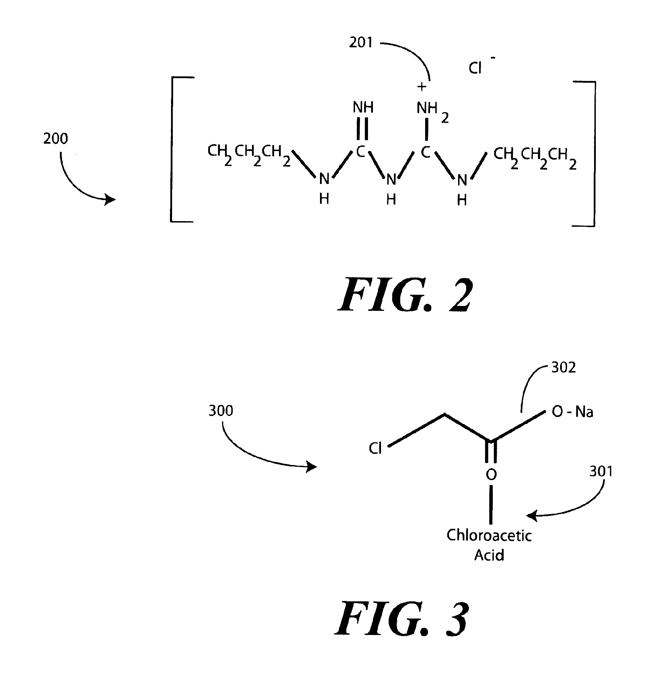

For example, one prior art solution is set forth in US Patent No. 6,369,289 to Orr, entitled "Method and Manufacture of a Wound Dressing for Covering an Open Wound." This prior art solution employs PHMB as an antimicrobial agent. As shown in FIG. 2, PHMB 200 has multiple positive charges 201. As such, the positive charges 201 of the PHMB 200 are able to form ionic bonds with the carboxylate groups (104) of the cellulose polymer chain (100).

The problem with the prior art of the '289 patent is twofold: First, there are a limited number of carboxylate groups (104) on the cellulose polymer chain (100) to which the PHMB 200 can bond. For example, cotton gauze processed with typical textile processing techniques may include carboxylate group bonding levels of between 10 to 40 mmol per kilogram of cotton. This level can be referred to as a "characteristic density of carboxylate bonding groups" associated with the cotton. Where the density is the characteristic density, roughly nine percent of the surface area of the gauze will contain carboxylate groups (COO-). As such, there is a limited amount of PHMB 200 that can be bond to the cellulose polymer chain through those carboxylate groups. For example, dressings made in accordance with the '289 patent may have a low percentage of PHMB 200, such as between 0.2 and 0.3 percent. Application of any more PHMB 200 results in the PHMB 200 leaching from the dressing to the patient, thereby irritating the patient's skin.

The second problem is that these low levels of PHMB 200 are frequently insufficient to kill some bacteria. It is known that low levels of PHMB 200 have a limited biocidal effect on bacteria. For instance, pseudomonas aeruginosa has an indicative MIC value of 100 ppm. Further, aspergillus niger has an indicative MIC value of 750 ppm. Gauze with only 0.2 or 0.3 percent PHMB 200 may be ineffective against these organisms.

Embodiments of the present invention provide a wound dressing having a density of carboxylate bonding groups that is greater than the characteristic density of carboxylate bonding groups. This is accomplished by applying a bonding agent to the dressing. In one embodiment, the bonding agent is one or more of chloroacetic acid or chlorocitric acid. This bonding agent bonds with the cellulose fiber of the dressing or the cellulose polymer chain (100). In so doing, the available number of carboxylate bonding groups suitable for bonding to an antimicrobial agent like PHMB 200 increases.

The bonding agent reacts with the hydroxyl groups of the cellulose fiber to form an ether linkage. Turning now to FIG. 3, illustrated therein is such an ether linkage 300. Specifically, a portion 302 of a cellulose fiber is shown bonded with a bonding agent molecule 301. In the illustrative embodiment of FIG. 3, chloroacetic acid is used as the bonding agent, although chlorocitric acid could have also been used. The bonding agent is joined to the bonding agent molecule 301 as an ether group.

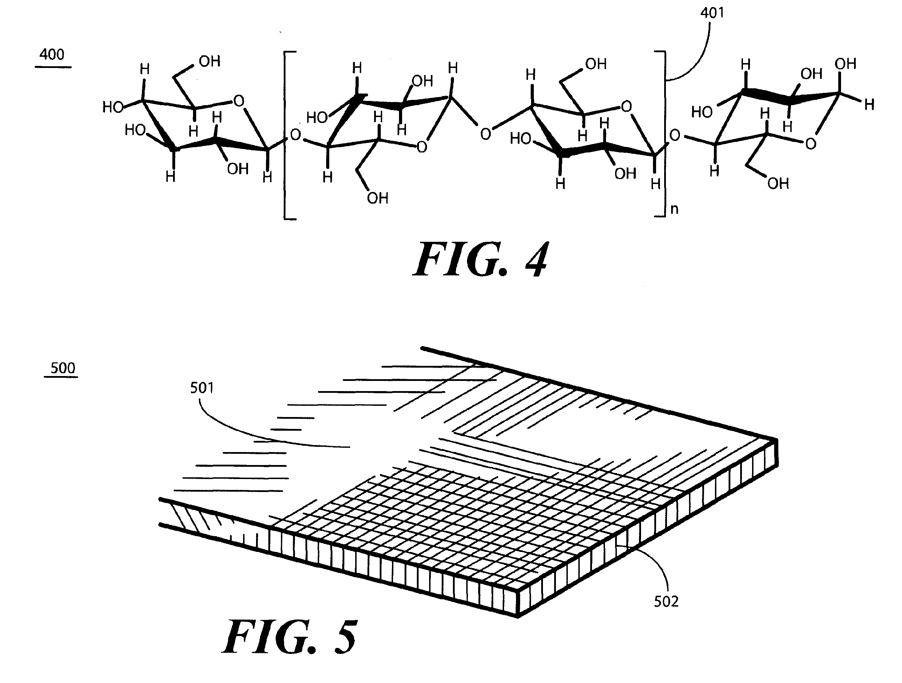

Turning now to FIG. 4, illustrated therein is an illustration of a cellulose fiber 400, such as that used in a flexible, absorbent layer of material in a wound dressing. The cellulose fiber 400 has been treated with the bonding agent molecule (301). As shown in FIG. 4, a number of carboxylate bonding groups, e.g., carboxylate bonding group 401, have been formed by the treatment of the cellulose fiber 400 with the bonding agent. The quantity of carboxylate bonding groups is significantly increased over the characteristic density of carboxylate bonding groups found in cellulose fiber that is untreated with the bonding agent. For example, while the characteristic density of carboxylate bonding groups may range from 10 to 40 mmol/kg in untreated cotton, resulting in an estimated 9% of the surface containing carboxylate bonding groups, in accordance with embodiments of the invention this can be increased the level to between 9 and 100 by controlling the treatment conditions such as bonding agent concentration, application temperature, application acidity level, and so forth. The high levels of carboxylate bonding groups available on the treated cellulose fiber are capable of ionically bonding to more PHMB (200) than can be accomplished with prior art methods. This increase of PHMB (200) increases the resulting wound dressing's antimicrobial efficacy. Further, the increased ionic bonding permits more PHMB (200) to be added without risk of leaching. This prevents skin irritation for the patient.

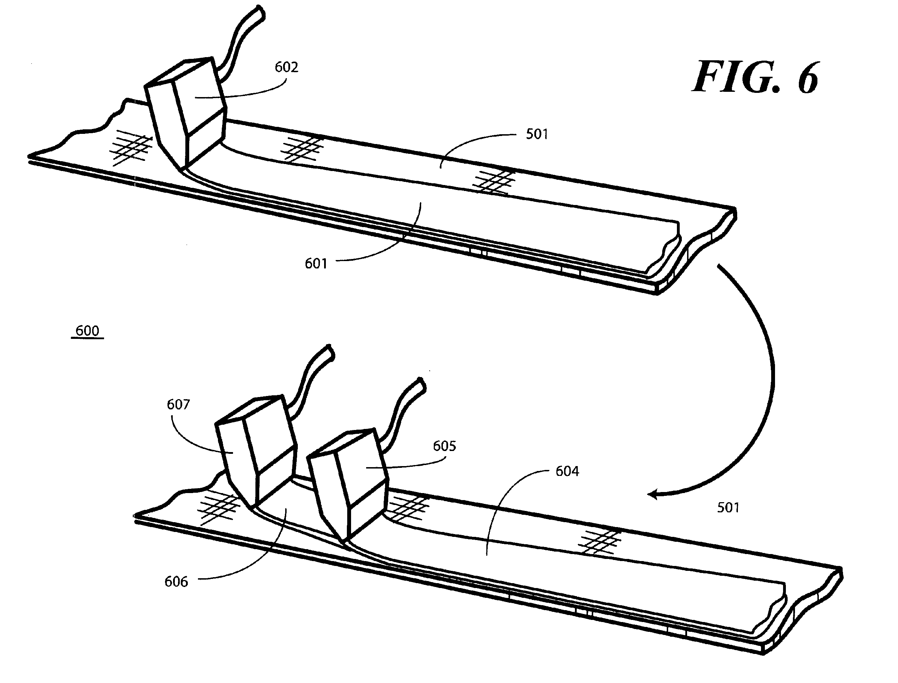

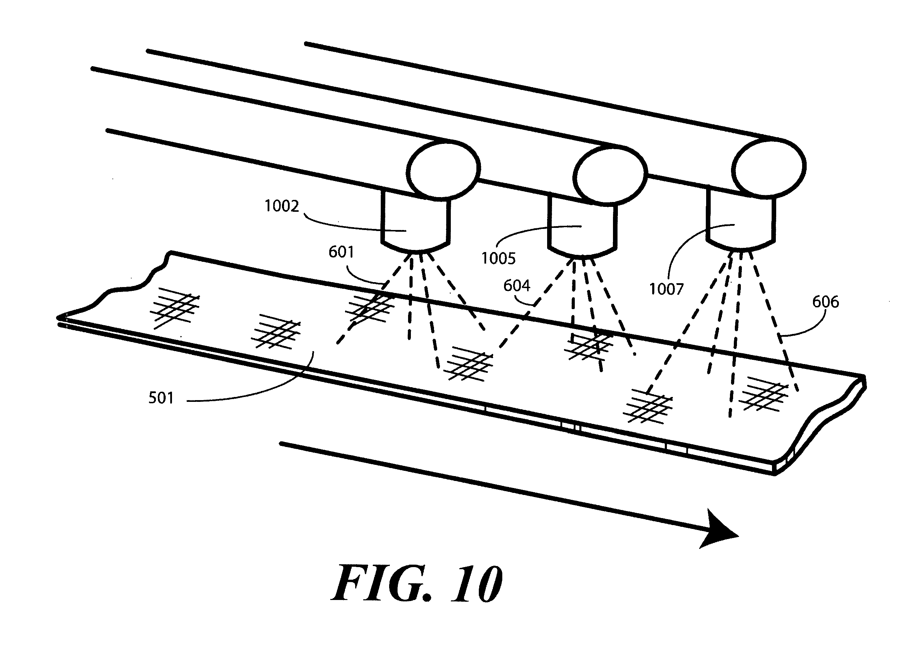

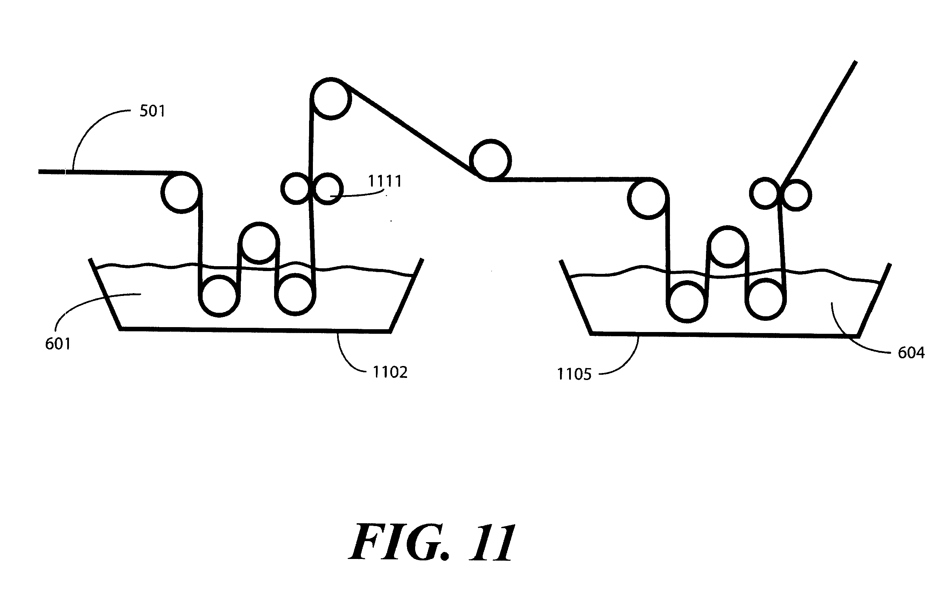

Turning now to FIGS. 5 and 6, illustrated therein is one embodiment of a wound dressing 500 in accordance with embodiments of the invention (FIG. 5) and a view of one process 600 by which a wound dressing can be made (FIG. 6). Alternative processes by which a wound dressing can be made in accordance with embodiments of the invention are illustrated in FIGS. 10 and 11, and will be described in more detail below.

In one embodiment, the wound dressing 500 includes a flexible layer of material 501 comprising a cellulose fiber 502. In one embodiment, the cellulose fiber is cotton or another organic material configured to be soft, pliable, and absorbent so as to absorb fluid emitted from a wound. In one embodiment, the wound dressing 500 is between fifty and one-hundred percent cellulostic.

The wound dressing 500 is treated with, and thus includes, a bonding agent 601. The bonding agent 601, in one embodiment, is an acidic agent, such as chloroacetic acid of chlorocitric acid. As shown in FIG. 6, the flexible layer of material 501 can be fed in sheet form under an applicator 602 capable of applying the bonding agent 601 to the flexible layer of material 501 in liquid form at a predetermined concentration. A sufficient amount of bonding agent 601 is applied to provide carboxylate bonding groups across between ten and eighty percent of the surface area of the flexible layer of material 501. The percentage of surface area covered with the bonding agent can be selected based upon a particular medical application or desired antimicrobial effect. It will be obvious to those of ordinary skill in the art having the benefit of this disclosure that the amount of bonding agent 601 applied will vary with the particular material used as the flexible layer of material 501, the size of the sheet to be coated, and the type and amount of cellulose fiber comprised therein. The application of the bonding agent 601, as well as the application of other components, step can occur at predetermined temperature, pressure, or acidity.

As set forth above, the cellulose molecules of the cellulose fiber 502 are composed of cellobiose units joined together by 1,4-β glycosidic bonds. The application of the bonding agent 601 increases the available number of carboxylate bonding groups, in one embodiment, between 50 and 360 mmol per kilogram of cellulose fiber 502. The bonding agent 601 reacts with the hydroxyl groups of the cellulose fiber molecules to form an ether linkage. By controlling the amount of bonding agent 601 applied, the degree of carboxylation can be controlled.

The flexible layer of material 501 can then be fed in sheet form under another applicator 605 capable of applying the antimicrobial agent 604 to the now treated flexible layer of material 501. The antimicrobial agent 604, which in one embodiment is PHMB, can be applied in liquid form at a predetermined concentration. The amount of antimicrobial agent 604 that can be absorbed will be directly proportional to the amount of bonding agent 601 that was applied, as the addition of more bonding agent 601 causes more carboxylation. The antimicrobial agent 604 ionically bonds to the carboxylate bonding groups provided by the bonding agent 601, and thereby bonds to the treated cellulose fiber 502 of the flexible layer of material 501. In one embodiment, the bonding agent 601 provides a carboxylate bonding group level of between 3000 and 25000 ppm.

In one embodiment of the invention, the flexible layer of material 501 further includes an antifungal agent 606, such as citric acid or undecylenic acid. As with the antimicrobial agent 604, the antifungal agent 606 can be applied by an applicator 607 in liquid form. Alternatively, the antifungal agent 606 can be combined with the antimicrobial agent 604 prior to application to the flexible layer of material 501. In such an embodiment, the combined antifungal agent 606 and antimicrobial agent 604 may be applied with a single applicator. In either case, this antifungal agent 606, where used, can increase the effectiveness of the wound dressing as an agent of biocide.

A sufficient amount of bonding agent 601 is applied to provide carboxylate bonding groups across between ten and eighty percent of the surface area of the flexible layer of material 501. The percentage of surface area covered with the bonding agent can be selected based upon a particular medical application or desired antimicrobial effect. It will be obvious to those of ordinary skill in the art having the benefit of this disclosure that the amount of bonding agent 601 applied will vary with the particular material used as the flexible layer of material 501, the size of the sheet to be coated, and the type and amount of cellulose fiber comprised therein.

An alternative method by which a wound dressing can be manufactured in accordance with embodiments of the invention is shown in FIG. 10. Turning briefly to FIG. 10, rather than the applicators (602,605,607) of FIG. 6, a plurality of sprayers 1002,1005,1007 can be used to apply the bonding agent 601, the antimicrobial agent 604, and the optional antifungal agent 606. In such a configuration, the flexible layer of material 501 can be fed in sheet form under the sprayers 1002,1005,1007, which spray the bonding agent 601 and the antimicrobial agent 604 onto the flexible layer of material 501. Each of these liquids can be applied at a predetermined concentration. As with the embodiment of FIG. 6, the antifungal agent 606 and antimicrobial agent 604 can be combined and applied by a single sprayer.

It may be the case in high volume scenarios that application of the bonding agent 601, the antimicrobial agent 604 and (where used) the antifungal agent 606 is more suited to a bulk application process. Turning briefly to FIG. 11, illustrated therein is a process in which the flexible layer of material 501 is fed through a bath 1102 containing the bonding agent 601. The flexible layer of material 501 can then be squeezed of excess bonding agent by a roller 1111 and fed into a second bath 1105 containing the antimicrobial agent 604, or a combination of the antimicrobial agent 604 and the antifungal agent (606). Optionally, a third bath of antifungal agent (606) alone may be used to apply the antifungal agent (606).

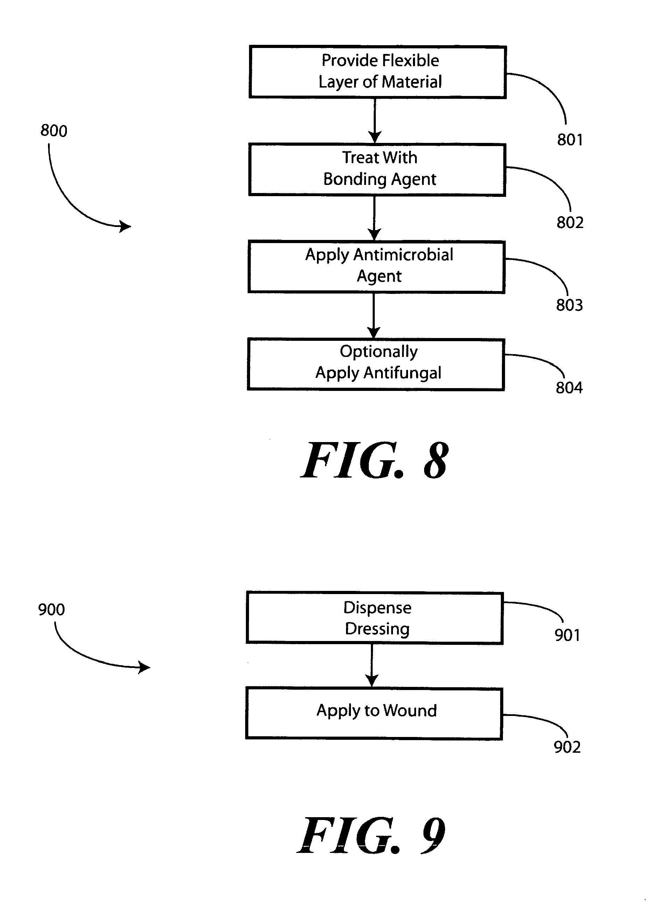

Turning now to FIG. 8, illustrated therein is a method 800 of manufacturing a wound dressing (500) in accordance with embodiments of the invention illustrated in a flow-chart configuration. At step 801, the flexible layer of material (501) that includes a cellulose fiber 502 is provided. At step 802, the flexible layer of material (501) is treated with a bonding agent (601) comprising an acid. In one embodiment, the flexible layer of material (501) is treated such that a resulting carboxylate bonding group level along the flexible layer of material (501) is between 50 and 360 milimol per kilogram of the cellulose fiber (502).

At step 803, the antimicrobial agent (604) is applied. In one embodiment, the antimicrobial agent (604) is applied such that the antimicrobial agent (604) ionically bonds with carboxylate bonding groups of the bonding agent (601). In one embodiment, the antimicrobial agent (604) is applied agent until it bonds to the cellulose fiber at a concentration of between 3000 and 25000 ppm. At optional step 804, an antifungal agent (606) is applied to the flexible layer of material (501).

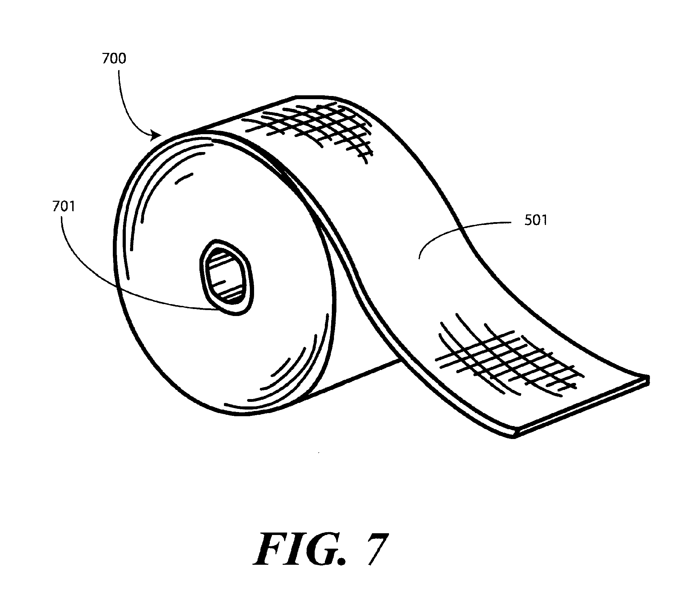

Turning now to FIG. 7, illustrated therein is a completed roll 700 of a wound dressing in accordance with embodiments of the invention. The roll 700 includes the flexible layer of material 501 that has been treated with the bonding agent (601), the antimicrobial agent (604), and optionally the antifungal agent (606). The flexible layer of material 501 is wrapped about a spool 701 such that a health care provider can conveniently access the flexible layer of material 501. The health care provider can then cut a sufficient amount of the flexible layer of material 501 to treat a wound on a patient. It will be clear to those of ordinary skill in the art having the benefit of this disclosure that the flexible layer of material 501 can be used in conjunction with other wound dressings, such as gauze patches, sutures, and so forth, in addition to being used on its own.

Turning now to FIG. 9, illustrated therein is a method 900 of treating a wound in accordance with embodiments of the invention illustrated in a flow-chart configuration. At step 901, an amount of wound dressing (500) is dispensed or cut. In one embodiment, the amount dispensed is sufficient to treat the wound, and to provide antimicrobial benefits to both the wound and areas surrounding the wound. At step 902, the wound dressing (500) is applied to the wound.

In the foregoing specification, specific embodiments of the present invention have been described. However, one of ordinary skill in the art appreciates that various modifications and changes can be made without departing from the scope of the present invention as set forth in the claims below. Thus, while preferred embodiments of the invention have been illustrated and described, it is clear that the invention is not so limited. Numerous modifications, changes, variations, substitutions, and equivalents will occur to those skilled in the art without departing from the spirit and scope of the present invention as defined by the following claims. Accordingly, the specification and figures are to be regarded in an illustrative rather than a restrictive sense, and all such modifications are intended to be included within the scope of present invention. The benefits, advantages, solutions to problems, and any element(s) that may cause any benefit, advantage, or solution to occur or become more pronounced are not to be construed as a critical, required, or essential features or elements of any or all the claims.

## Claims

1. A wound dressing, comprising:
a flexible layer of material comprising a cellulose fiber and configured to absorb fluid from a wound;
a bonding agent comprising an acid, applied to the flexible layer of material; and
an antimicrobial agent;
wherein the boding agent ionically bonds the antimicrobial agent to the cellulose fiber.

2. The wound dressing of claim 1, wherein the bonding agent ionically bonds the antimicrobial agent to the cellulose fiber by bonding the antimicrobial agent to carboxylate groups of the bonding agent.

3. The wound dressing of claim 2, the flexible layer of material, when untreated with the bonding agent, has a characteristic density of carboxylate bonding groups suitable for bonding to the antimicrobial agent associated therewith, wherein treatment with the bonding agent provides more carboxylate bonding groups suitable for bonding to the antimicrobial agent along the flexible layer of material than the characteristic density of carboxylate bonding groups.

4. The wound dressing of claim 3, wherein the characteristic density of carboxylate bonding groups is less than nine percent of a surface area of the cellulose fiber.

5. The wound dressing of claim 3, wherein the bonding agent provides carboxylate bonding groups across between ten percent and eighty percent of a surface area of the cellulose fiber.

6. The wound dressing of any preceding claim, further comprising an antifungal agent applied to the flexible layer of material.

7. A method of manufacturing a wound dressing, the method comprising:
providing a flexible layer of material comprising a cellulose fiber;
treating the flexible layer of material with a bonding agent comprising an acid such that a resulting carboxylate bonding group level along the flexible layer of material of between 50 and 360 milimol per kilogram of the cellulose fiber; and
applying an antimicrobial agent such that the antimicrobial agent ionically bonds with carboxylate bonding groups of the bonding agent.

8. The wound dressing of any one of claims 1-6 or the method of claim 7, wherein the bonding agent comprises at least one of chloroacetic acid or chlorocitric acid.

9. The wound dressing of claim 8 or the method of claim 8, wherein the antimicrobial agent comprises polyhexamethylene biguanide.

10. The wound dressing of claim 9, the bonding agent provides a carboxylate bonding group level of between 50 and 360 milimol per kilogram of the cellulose fiber.

11. The wound dressing of claim 9, wherein the wound dressing comprises a concentration of the polyhexamethylene biguanide of between 3000 and 25000 ppm.

12. The wound dressing of claim 9, wherein the flexible layer of material is between fifty and one hundred percent cellulostic.

13. The wound dressing of claim 9, wherein the cellulose fiber comprises cotton.

14. The method of claim 9, wherein the applying the antimicrobial agent comprises applying the antimicrobial agent until the polyhexamethylene biguanide bonds to the cellulose fiber at a concentration of between 3000 and 25000 ppm.

15. The method of claim 14, further comprising applying an antifungal agent to the flexible layer of material.

16. The wound dressing of claim 6 or the method of claim 15, wherein the antifungal agent comprises at least one of citric acid or undecylenic acid.
